# DEMANDE DE BREVET EUROPEEN

(11) **EP 0 811 678 A1**
(43) Date de publication de la demande: **10.12.1997**
(21) Numéro de dépôt: 96201590.5
(22) Date de dépôt: 08.06.1996
(51) Int. Cl.: C11B 1/00, C11B 5/00, A23D 9/06, A61K 7/00

(54) **Extraction d'antioxydants**

(71) Demandeur: SOCIETE DES PRODUITS NESTLE S.A., 1800 Vevey (CH)
(72) Inventeur: Aeschbach, Robert, 1800 Vevey (CH); Bracco, Umberto, 1800 Vevey (CH); Rossi, Patricia, 1814 La Tour-de-Peilz (CH)
(74) Mandataire: Archambault, Jean

(57) **Abrégé**

L'invention concerne un procédé d'extraction d'antioxydants d'olive, une fraction lipidique enrichie en antioxydants, un extrait enrichi en antioxydants ainsi qu'une composition alimentaire ou cosmétique, comprenant cet extrait.

## Description

La présente invention a pour objet a procédé d'extraction d'antioxydants d'olive, une fraction lipidique enrichie en antioxydants, un extrait enrichi en antioxydants ainsi qu'une composition alimentaire ou cosmétique, contenant cette fraction lipidique ou de cet extrait.

Dans un procédé classique, les olives sont traitées par pression et l'on obtient trois phases, une phase aqueuse, une phase lipidique et une phase solide. Dans un tel procédé, la phase aqueuse et la phase solide sont éliminées. Des antioxydants hydrosolubles sont ainsi perdus dans la phase solide et également dans la phase aqueuse. De plus, ces antioxydants sont tellement dilués dans la phase aqueuse, que, même si on le souhaitait, l'on ne pourrait plus les récupérer.
Par ailleurs, la phase aqueuse, dont le volume est approximativement quatre fois supérieur au volume de la phase lipidique, doit être traitée en station d'épuration à titre d'eaux usées.

Ainsi, A. Uzzan (Manuel des corps gras - ISBN 2 - 85206 - 662/9 - 1992 - 763-768) décrit notamment un procédé d'obtention de l'huile d'olive par pressage, dans lequel les olives sont nettoyées, malaxées puis passées dans une presse hydraulique, de manière à séparer la phase liquide et la phase solide. A ce stade, la phase liquide est divisée par décantation ou par centrifugation en ses deux constituants, la phase aqueuse contenant les substances hydrosolubles de l'olive et l'huile d'olive. Ces deux constituants sont encore une fois centrifugés, de manière, d'une part, à recueillir l'huile clarifiée et purifiée et, d'autre part, à extraire l'huile résiduelle contenue dans la phase aqueuse. Cette phase aqueuse ainsi que la phase solide précédente, encore riches en antioxydants, sont éliminées.

La présente invention a pour but de proposer un procédé permettant de recueillir, à partir d'olives vertes et/ou d'olives mûries, c'est à dire à divers degrés de maturité, d'une part une fraction lipidique enrichie en antioxydants et d'autre part un extrait enrichi en antioxydants.

A cet effet, dans le procédé d'extraction d'antioxydants d'olive selon la présente invention:
- on broie des olives,
- on sèche sous vide ces olives broyées, de manière à obtenir des olives séchées riches en antioxydants hydrosolubles,
- on effectue uni pressage de ces olives séchées, de manière à recueillir une fraction lipidique enrichie en antioxydants et un tourteau,
- on effectue sur le tourteau au moins une extraction à chaud au MCT, c'est à dire un mélange de triglycérides à chaine moyenne, ou avec a alkylène glycol en C₂-C₆ par pression supérieure ou égale à 40 bar,
- puis l'on recueille a extrait enrichi en antioxydants à partir du tourteau.

On a constaté avec surprise qu'un tel procédé permet effectivement d'obtenir une fraction lipidique et un extrait enrichis en antioxydants et, plus particulièrement, en antioxydants hydrosolubles.
De plus, le procédé selon la présente invention, du fait de l'absence d'eaux usées, présente des avantages écologiques évidents.

On peut choisir les olives parmi les olives vertes et/ou les olives mûries, par exemple.

On peut congeler les olives, de manière à faciliter le broyage, par exemple.

On broie donc les olives. Pour ce faire, on peut utiliser des techniques usuelles pour le broyage des fruits à noyaux, notamment des moulins à marteaux, à disques, colloïdaux, à molasses ou a cutter à lames.

On peut traiter enzymatiquement les olives broyées, à l'aide d'enzymes d'origine bactérienne ou fongique, d'hydrolases, de glucosidases ou de polyphénolhydrolases, par exemple, de manière à hydrolyser les glycosides et améliorer l'extraction des antioxydants, par exemple.

On peut sécher sous vide les olives broyées à une température inférieure ou égale à 80° C, de manière à recueillir des olives séchées riches en antioxydants hydrosolubles et dont la teneur en eau est de 1-20% en poids, par exemple. De préférence, on sèche, de manière à obtenir des olives séchées dont la teneur en eau est de 5-10% en poids. On favorise, ainsi, uniquement la formation de deux phases, la fraction lipidique enrichie en antioxydants et le tourteau, lors de l'étape de pressage.

On peut sécher par lyophilisation à une pression réduite de 10⁻³-10⁻¹ bar ou dans une étuve à vide à une pression réduite de 0,1-0,2 bar, par exemple.

On peut préchauffer les olives séchées et les maintenir à chaud pendant une certaine durée, avant d'effectuer le pressage, de manière à augmenter la teneur en antioxydants de la fraction lipidique.

On effectue donc un pressage sur ces olives séchées, de manière à recueillir une fraction lipidique enrichie en antioxydants et a tourteau. On peut effectuer ce pressage à température ambiante ou à chaud dans une presse à piston munie d'une cage filtrante, notamment une presse de type Carver commercialisée par la société Fred S. Carver, Menomonee Falls, Wisconsin - USA, de manière à presser et à filtrer en une seule étape.

Puis, on effectue donc sur le tourteau au moins une extraction à chaud au MCT ou avec un alkylène glycol en C₂-C₆ par pression supérieure ou égale à 40 bar. On peut effectuer sur le tourteau au moins une extraction dans un rapport pondéral MCT ou alkylène glycol en C₂-C₆/ tourteau de 0,5 à 2, par exemple. On peut effectuer cette ou ces extractions à chaud dans une presse à piston munie d'une cage filtrante, de type Carver.
L'alkylène glycol peut être le glycol, le 1,2-propylène glycol ou le 1,3-butylène glycol, par exemple.
Lors d'une extraction à chaud au MCT par pression, on isole principalement les antioxydants liposolubles et lors d'une extraction à chaud avec un alkylène glycol en C₂-C₆ par pression, on isole les antioxydants liposolubles et les antioxydants hydrosolubles.

La présente invention concerne également une fraction lipidique enrichie en antioxydants dont le temps d'induction est de 15-75 h à une température de 110- 120° C.

De plus, la présente invention concerne a extrait enrichi en antioxydants susceptible d'être obtenu par la mise en oeuvre dudit procédé.

Cet extrait comprend des antioxydants hydrosolubles, notamment l'hydroxytyrosol, le tyrosol, des acides phénoliques et l'oleuropéine.

La présente invention concerne une composition alimentaire ou cosmétique, comprenant 0,5-4% d'extrait enrichi en antioxydants obtenu par la mise en oeuvre du procédé selon la présente invention.

Enfin, la présente invention concerne une composition alimentaire ou cosmétique, comprenant une fraction lipidique enrichie en antioxydants, obtenue par la mise en oeuvre du procédé selon la présente invention.

Le procédé selon la présente invention est décrit plus en détails dans les exemples non limitatifs ci-après. Dans ces exemples, les pourcentages sont donnés en poids, sauf indication contraire.

### Exemple 1

On broie à l'aide du broyeur Alpina, commercialisé par la société C. Hoegger et Cie AG, Gossau, CH- St Gall, 1 kg d'olives vertes congelées dont la teneur globale en eau est de 55% en poids.

On sèche ces olives vertes broyées dans une étuve sous vide de type Inox Maurer 20, commercialisée par la société Inox Maurer AG, Trimbach, CH - Soleure, à une température de 55° C et à une pression réduite de 0,1 bar, de manière à obtenir des olives vertes séchées dont la teneur globale en eau est de 6% en poids.

Puis, l'on effectue un pressage à température ambiante sur ces olives vertes séchées dans une presse à piston, de type Carver, à 500 bar pendant 60 min, de manière à recueillir la fraction lipidique enrichie en antioxydants et le tourteau.

Puis, l'on effectue sur 50 g du tourteau ainsi recueilli une extraction à chaud au 1,2 propylène glycol.
Pour ce faire, aux 50 g de tourteau on ajoute 50 g de 1,2-propylène glycol. On laisse le tout sous agitation pendant 60 min à 80° C et l'on presse le mélange dans une presse à piston, de type Carver, à 500 bar pendant 60 min.

On recueille ainsi l'extrait enrichi en antioxydants. Le test Rancimat ® à 110° C dans différentes graisses et huiles donne le pouvoir antioxydant sous forme d'indices antioxydants indiqués dans le tableau I ci-après.
L'indice antioxydant est défini comme étant le rapport:
temps d'induction (échantillon: extrait + huile) / temps d'induction (huile).

### Test d'oxydation Rancimat® à 110° C:

On place l'échantillon dans un réacteur fermé.
L'échantillon est chauffé à 110° C et est saturé en oxygène provenant de l'air introduit dans le réacteur.
Durant l'oxydation, le réacteur est lui-même relié par un tube souple à un récipient contenant de l'eau distillée et où est plongée une électrode de platine.
Les composés volatiles entraînent une augmentation de la conductivité.
La conductivité est mesurée et les périodes d'induction sont calculées.
On détermine le temps d'induction graphiquement à partir de la courbe transcrite de la conductivité en fonction du temps par intersection de la tangente à la courbe avec l'axe des temps.

### Exemple comparatif i

On procède de la manière telle que décrite à l'exemple 1, à l'exception du fait que l'on effectue sur 50 g du tourteau recueilli une extraction à chaud par voie organique, à l'éthanol à 85%.

Pour ce faire, aux 50 g de tourteau on ajoute 100 ml d'éthanol à 85%. On laisse le tout sous agitation pendant 60 min à 80° C et l'on filtre, avant de concentrer à 50% de volume. Puis, on ajoute 50 g de propylèneglycol, on évapore l'éthanol et l'on centrifuge pendant 10 min à 3000 rpm, pour clarifier.

On recueille un extrait contenant les antioxydants. Le test Rancimat® à 110° C dans différentes graisses et huiles donne le pouvoir antioxydant sous forme d'indices antioxydants indiqués dans le tableau I ci-après.

**Tableau I**

| Pour le test Rancimat ®, les mesures sont effectuées en présence de 2% d'extrait par rapport à la graisse de poule ou par rapport à l'huile d'olive. | | |
|---|---|---|
| | Test Rancimat ® / 110° C (indice antioxydant) | |
| Exemples | huile d'olive | graisse de poule |
| 1 | 3,8 | 8,6 |
| i | 4,5 | 9,9 |

Les mesures du pouvoir antioxydant, indiquées sous forme d'indice antioxydant, au tableau I mettent en évidence la qualité et la stabilité oxydative de l'extrait obtenu par la mise en oeuvre par le procédé selon la présente invention (exemple 1). Cette qualité et cette stabilité oxydative sont comparables à celles que l'on a pour un extrait obtenu par la mise en oeuvre d'un procédé plus complexe, dans lequel on effectue une extraction à chaud par voie organique (exemple i).

### Exemple 2

On procède de la manière décrite à l'exemple 1, à l'exception du fait que l'on effectue les mesures pour le test Ranicimat ® en présence de 1% d'extrait par rapport à la graisse de poule ou par rapport à l'huile d'olive.

Par ailleurs, on mesure le pouvoir antioxydant sous forme d'un indice antioxydant par la méthode d'électrode à oxygène à 30° C dans de l'huile de maïs. On effectue ces mesures en présence de 1% d'extrait par rapport à l'huile de maïs.

De plus, on mesure par le test Rancimat® à 120° C, le temps d'induction de la fraction lipidique recueillie après le pressage à température ambiante des olives vertes séchées. La valeur du temps d'induction de la fraction lipidique est mentionnée au tableau III ci-après.

### Test d'oxydation: électrode à oxygène à 30° C.

On prépare une émulsion en mélangeant 5 % d'huile et le % indiqué en antioxydants par rapport à l'huile dans une solution tampon de pH 7( No.9477, Merck, Darmstadt, D) avec 0,1 % d'émulsifiant par agitation vigoureuse sous azote pendant 30 min que l'on émulsifie par 6 passages consécutifs à 30° C dans un microfluidiseur H 5000.
On mesure ensuite la stabilité oxydative de l'émulsion à l'aide d'une électrode TRI OX EO 200® couplée avec un oxygène-mètre OXI 530®.
On attend 5 à 10 min jusqu'à ce que le pourcentage de saturation d'oxygène ait me valeur constante.
Cette mesure s'effectue à 30° C sous agitation en vase clos, après adjonction de 5 ml du catalyseur Hemin (Fluka AG, Buchs, CH) à 100 ml d'émulsion. Le catalyseur Hemin est préparé à partir de 52 mg d'Hemin dans 100 ml d'eau, auxquels on ajoute 8 gouttes de KOH à 10 %.
Le temps d'induction représente la durée en heures pour une absorption totale de l'oxygène dissout.

Le test Rancimat® à 110° C dans différentes graisses et huiles et la méthode d'électrode à oxygène à 30° C dans l'huile de maïs donnent le pouvoir antioxydant sous forme d'indices antioxydants indiqués dans le tableau II ci-après.

### Exemple comparatif ii

On procède de la manière décrite à l'exemple 2, à l'exception du fait que l'on ne sèche pas les olives vertes broyées.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

La valeur du temps d'induction à 120° C de la fraction lipidique est mentionnée au tableau III ci-après.

### Exemple 3

On procède de la manière telle que décrite à l'exemple 1, à l'exception du fait que l'on préchauffe à 70° C pendant 60 min les olives séchées avant d'effectuer le pressage.

Après refroidissement à température ambiante, on effectue le pressage à température ambiante dans une presse à piston, de type Carver®, à 500 bar pendant 60 min. On recueille ainsi la fraction lipidique enrichie en antioxydants et le tourteau.

On mesure, par le test Rancimat® à 120° C, le temps d'induction de la fraction lipidique. La valeur du temps d'induction de la fraction lipidique est mentionnée au tableau III ci-après.

Par ailleurs, on effectue sur 50 g du tourteau recueilli une extraction à chaud au 1,2 propylène glycol.
Pour ce faire, aux 50 g de tourteau on ajoute 50 g de 1,2-propylène glycol. On laisse le tout sous agitation pendant 60 min à 80° C et l'on presse le mélange dans une presse à piston, de type Carver, à 500 bar pendant 60 min.

On recueille l'extrait enrichi en antioxydants.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

### Exemple 4

On procède de la manière décrite à l'exemple 2, à l'exception du fait que l'on effectue sur 50 g du tourteau recueilli une extraction à chaud au MCT.

Pour ce faire, aux 50 g de tourteau on ajoute 50 g de MCT. On laisse le tout sous agitation pendant 60 min à 80° C et l'on presse le mélange dans une presse à piston, de type Carver, à 500 bar pendant 60 min.

On recueille l'extrait enrichi en antioxydants.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

### Exemple comparatif iv

On procède de la manière décrite à l'exemple 4, à l'exception du fait que l'on ne sèche pas les olives vertes broyées.

Le test Rancimat® à 110° C, dans la graisse de poule ou dans l'huile d'olive et la méthode d'électrode à oxygène à 30° C dans l'huile de maïs donnent le pouvoir antioxydant sous forme d'indices antioxydants indiqués dans le tableau II ci-après.

### Exemple 5

On procède de la manière décrite à l'exemple 4, à l'exception du fait que l'on préchauffe à 70° C pendant 60 min les olives vertes séchées avant d'effectuer le pressage.

Après refroidissement à température ambiante, on effectue le pressage à température ambiante dans une presse à piston, de type Carver®, à 500 bar pendant 60 min. On recueille ainsi la fraction lipidique enrichie en antioxydants et le tourteau.

On mesure par le test Rancimat® à 120° C, le temps d'induction à partir de 1% de fraction lipidique ainsi recueillie.

Par ailleurs, on effectue sur 50 g du tourteau recueilli une extraction à chaud au MCT.
Pour ce faire, aux 50 g de tourteau on ajoute 50 g de MCT. On laisse le tout sous agitation pendant 60 min à 80° C et l'on presse le mélange dans une presse à piston, de type Carver, à 500 bar pendant 60 min.

On recueille l'extrait enrichi en antioxydants.

Le test Rancimat® à 110° C, dans la graisse de poule ou dis l'huile d'olive et la méthode d'électrode à oxygène à 30° C dans l'huile de maïs donnent le pouvoir antioxydant sous forme d'indices antioxydants indiqués dans le tableau II ci-après.

**Tableau II**

| On effectue toutes les mesures en présence de 1% d'extrait par rapport à l'huile de maïs, par rapport à la graisse de poule ou par rapport à l'huile d'olive. | | | |
|---|---|---|---|
| | Test Rancimat ® / 110°C (indice antioxydant) | | Test électrode à oxygène / 30°C (indice antioxydant) |
| Exemples | graisse de poule | huile d'olive | huile de maïs |
| 2 | 4,3 | 2,1 | 7,8 |
| ii | 1,8 | 1,1 | 1,5 |
| 3 | 3,9 | 1,6 | 8,4 |
| 4 | 3,2 | 1,3 | - |
| iv | 1,2 | 1 | - |
| 5 | 1,9 | 1,2 | - |
| -: non testé | | | |

Les mesures du pouvoir antioxydant, indiquées sous forme d'indice antioxydant, respectivement aux exemples 2 et ii et aux exemples 4 et iv dans le tableau II, mettent en évidence une augmentation de la stabilité oxydative de l'extrait enrichi en antioxydants, obtenu à partir du tourteau, dans le cas où l'on sèche les olives vertes broyées.

De plus, les mesures du pouvoir antioxydant, indiquées sous forme d'indice antioxydant, respectivement aux exemples 2 et 3 et aux exemples 4 et 5 dans le tableau II, mettent en évidence le fait que si l'on effectue un pressage à chaud sur les olives vertes séchées, l'extrait enrichi en antioxydants, à partir du tourteau, présente une stabilité oxydative plus faible.

Enfin, les mesures du pouvoir antioxydant, indiquées sous forme d'indice antioxydant dans le test d'électrode à oxygène, aux exemples 2 et 3, mettent en évidence la qualité et la stabilité oxydative des composés antioxydants en émulsion, par rapport aux résultats obtenus dans une huile dans le test d'oxydation Rancimat®.

**Tableau III**

| Exemples | Test Rancimat ® / 120°C (temps d'induction en heures) |
|---|---|
| 2 | 25 |
| ii | 11 |
| 3 | 42 |

Les mesures du pouvoir antioxydant, indiquées sous forme de temps d'induction, dans le tableau III, mettent en évidence le fait que si l'on préchauffe les olives vertes séchées avant d'effectuer le pressage à température ambiante, la fraction lipidique enrichie en antioxydants présente une stabilité oxydative accrue.

### Exemple 6

On procède de la manière telle que décrite à l'exemple 2, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

### Exemple comparatif vi

On procède de la manière telle que décrite à l'exemple ii, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

### Exemple 7

On procède de la manière telle que décrite à l'exemple 4, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

### Exemple comparatif vii

On procède de la manière telle que décrite à l'exemple iv, à l'exception du fait que les olives sont des olives mûries.

Le test Rancimat® à 110° C, dans la graisse de poule et la méthode d'électrode à oxygène à 30° C dans l'huile de maïs donnent le pouvoir antioxydant sous forme d'indices antioxydants indiqués dans le tableau IV ci-après.

**Tableau IV**

| On effectue toutes les mesures en présence de 1% d'extrait par rapport à l'huile de maïs ou par rapport à la graisse de poule. | | |
|---|---|---|
| | Test Rancimat ® / 110°C (indice antioxydant) | Test électrode à oxygène / 30°C (indice antioxydant) |
| Exemple | graisse de poule | huile de maïs |
| 6 | 1,8 | 1,9 |
| vi | 1,2 | 1,7 |
| 7 | 1,1 | 1,3 |
| vii | 1 | 1,3 |

Les mesures du pouvoir antioxydant, indiquées sous forme d'indice antioxydant, respectivement aux exemples 6 et vi et aux exemples 7 et vii dans le tableau IV, mettent en évidence une augmentation de la stabilité oxydative de l'extrait enrichi en antioxydants, obtenu à partir du tourteau, dans le cas où l'on sèche les olives mûries et préalablement broyées.

### Exemple 8

On réalise la stabilisation d'une huile d'olive non vierge du commerce (olio Sasso ®) avec un extrait enrichi en antioxydants d'olive préparé conformément à l'exemple 1.

A 100 g d'huile d'olive, on ajoute 2 g de l'extrait et on obtient un huile 3,8 fois plus protégée que l'huile sans l'extrait par mesure du pouvoir antioxydant sous forme d'indice antioxydant dans le test Rancimat ® à 110° C.

## Revendications

1. Procédé d'extraction d'antioxydants d'olives, dans lequel:
- on broie des olives,
- on sèche sous vide ces olives broyées, de manière à obtenir des olives séchées riches en antioxydants hydrosolubles,
- on effectue un pressage de ces olives séchées, de manière à recueillir une fraction lipidique enrichie en antioxydants et un tourteau,
- on effectue sur le tourteau au moins une extraction à chaud au MCT ou avec un alkylène glycol en C₂-C₆ par pression supérieure ou égale à 40 bar,
- puis l'on recueille a extrait enrichi en antioxydants à partir du tourteau.

2. Procédé selon la revendication 1, dans lequel les olives sont des olives vertes et/ou des olives mûries.

3. Procédé selon la revendication 1, dans lequel les olives sont des olives congelées.

4. Procédé selon la revendication 1, dans lequel on sèche les olives broyées sous vide à une température inférieure ou égale à 80° C, de manière à obtenir des olives séchées riches en antioxydants hydrosolubles et dont la teneur globale en eau est de 1-20% en poids.

5. Procédé selon la revendication 1, dans lequel les olives séchées ont une teneur globale en eau de 5-10% en poids.

6. Procédé selon la revendication 1, dans lequel on effectue sur le tourteau au moins une extraction dans un rapport pondéral MCT ou alkylène glycol en C₂-C₆/ tourteau de 0,5 à 2.

7. Procédé selon la revendication 1, dans lequel l'alkylène glycol en C₂-C₆ est le glycol, le 1,2-propylène glycol ou le 1,3-butylène glycol.

8. Fraction lipidique enrichie en antioxydants dont le temps d'induction est de 15-75 h à 110-120° C.

9. Extrait enrichi en antioxydants susceptible d'être obtenu par la mise en oeuvre du procédé selon l'une des revendications 1 à 7.

10. Extrait enrichi en antioxydants selon la revendication 9, comprenant notamment des antioxydants hydrosolubles.

11. Composition alimentaire ou cosmétique, comprenant 0,5-4% en poids d'extrait enrichi en antioxydants obtenu par la mise en oeuvre du procédé selon l'une des revendications 1 à 7.

12. Composition alimentaire ou cosmétique, comprenant une fraction lipidique enrichie en antioxydants obtenue par la mise en oeuvre du procédé selon la revendication 1.
